# EUROPEAN PATENT APPLICATION

(11) **EP 3 903 591 A1**
(43) Date of publication of application: **03.11.2021**
(21) Application number: 19903418.2
(22) Date of filing: 24.12.2019
(51) Int. Cl.: A23C 9/13, A61K 31/7048, A61P 39/00, A23L 29/281, A23L 33/105, A23G 3/34, A23G 4/12, A23L 2/52

(54) **HEAT STRESS ALLEVIATION AGENT**

(30) Priority: 28.12.2018 JP 2018247186
(71) Applicant: Hayashibara Co., Ltd., Okayama-shi, Okayama 702-8006 (JP)
(72) Inventor: HASHIMOTO Satoko, Okayama-shi Okayama 702-8006 (JP); YOSHIZANE Chiyo, Okayama-shi Okayama 702-8006 (JP); OGIHARA Saori, Okayama-shi Okayama 702-8006 (JP); ENDO Shin, Okayama-shi Okayama 702-8006 (JP); MIYAKE Masaki, Okayama-shi Okayama 702-8006 (JP); MITSUZUMI Hitoshi, Okayama-shi Okayama 702-8006 (JP)
(74) Representative: Daniels, Jeffrey Nicholas
(86) International application number: PCT/JP2019/050488
(87) International publication number: WO 2020/138026

(57) **Abstract**

The present invention addresses the problem of providing a novel heat stress relieving agent that can be taken easily, conveniently and safely even by infants and the elderly, who are susceptible to heat stress such as heat illness, that rapidly promotes sweating in a heat stress environment, and that prevents, alleviates, or improves symptoms and disorders caused by heat stress, such as heat illness. The above problem is solved by providing a heat stress relieving agent containing glycosyl hesperetin as an effective ingredient, and a basal body temperature maintaining agent containing glycosyl hesperetin as an effective ingredient.

## Description

### Technical Field

The present invention relates to a heat stress relieving agent, and more particularly, to a heat stress relieving agent which functions as a perspiration accelerator or a core body temperature rise suppressing agent under a heat stress environment, and a basal body temperature maintaining agent.

### Background Art

In recent years, due to global warming and the resulting increase in mid-summer days, extremely hot days, and tropical nights, as well as the urban heat island phenomenon, exposure to heat stress that directly affects the body has increased, and the incidence of heat stress-induced diseases such as heat illness has increased. The incidence of heat illness has been frequently caused by exercise and labor in a hot stress environment, but which has recently been increasing in daily life due to the effects of global warming and other factors. Heat illness refers to the marked deterioration of in vivo regulatory function due to inadequate heat dissipation, disruption of the balance of water and salt in the body under heat stress. Heat illness can be classified into heat cramps, heat exhaustion, and heatstroke with impaired thermoregulation depending on the symptoms, and can lead to death in severe cases.

Increase of heat illness patients, especially in people so-called susceptible to heat stress such as infants and children whose perspiring function is immature, and elderly people whose body temperature regulation function or the like, perspiring function are weakening due to aging, has been becoming a social problem, and effective heat stress countermeasures against the background of such a situation are desired.

Under heat stress environment, the living body secretes sweat from sweat gland tissue and releases it from the epidermis, and removes heat of vaporization when sweat evaporates, thereby releasing heat from the living body and regulating body temperature. In other words, diseases caused by heat stress, such as heat illness, can be prevented, alleviated, and ameliorated by regulating body temperature through the appropriate promotion of sweating.

As a countermeasure against heat illness, which is a disease caused by heat stress, it is recommended to take frequent hydration and salt intake in addition to devising a living environment and clothing, for example, the consumption of soft drinks containing a specific sugar level and sodium has been reported (Patent Document 1). In addition, as a countermeasure against heat illness, the regulation of body temperature by promoting perspiration has been attracting attention on focus of the decline in body temperature regulation due to ageing and the decrease in perspiration due to immaturity of the sweating function in infants and children. As a method to promote sweating, for example, it has been disclosed that perspiration is promoted by consuming beverages that contain non-polymeric catechins and have an osmotic pressure lower than that of body fluids (Patent Document 2). However, such non-polymeric catechins have a strong bitter taste and cannot be easily consumed every day, and which are generally considered to be unsuitable for infants and children because non-polymeric catechins contain a large amount of caffeine and have excitatory effects. In addition, a composition containing milk protein as an effective ingredient has been proposed as an ingestible perspiration-promoting composition in a heat stress environment (Patent Document 3). However, such milk proteins require a large intake of more than 30 g as a single intake, making it difficult to ingest them on a daily basis. A heat illness prevention agent characterized by the inclusion of an oil and fat composition containing α-linolenic acid has also been proposed (Patent Document 4), but the composition has the disadvantage of low solubility in water, making it difficult to easily formulate or process into food and beverage products such as sports drinks.

### Prior Art Document

### Patent Document

Patent Document 1: JP-A-2015-008712
Patent Document 2: JP-A-2014-019691
Patent Document 3: International Patent Publication WO2018-123873
Patent Document 4: JP-A-2016-037498

### SUMMARY OF THE INVENTION

### Problems to be Solved by the Invention

The present invention has been made in view of the above-mentioned prior art. The problem to be solved is to provide a new heat stress relieving agent which can be easily, conveniently and safely ingestible even by infants and the elderly who are susceptible to heat stress such as heat illness, and which promotes perspiration quickly in a heat stress environment to prevent, alleviate or improve symptoms and diseases such as heat illness caused by heat stress.

### Means for Solving the Problems

In order to solve the above-mentioned problems, the inventors have made intensive research efforts and unexpectedly found that oral intake of glycosyl hesperetin alleviates heat stress, and more specifically, that glycosyl hesperetin promotes perspiration and suppresses the increase in core body temperature under a heat stress environment, thereby completing the heat stress relieving agent of the present invention.

As a result of further extensive research efforts, the present inventors have newly found that a basal body temperature is maintained by orally taking glycosyl hesperetin unexpectedly, and which has completed the basal body temperature maintaining agent of the present invention.

That is, the present invention solves the above problems by providing a heat stress relieving agent containing glycosyl hesperetin as an effective ingredient and a basal body temperature maintaining agent containing glycosyl hesperetin as an effective ingredient.

Although it has been disclosed that hesperidin and its glycosides are the effective ingredients of antiperspirants for external use in Japanese Patent Application Publication S52-105221, an antiperspiration is the opposite effect to the perspiration promotion which is thought to be effective in relieving heat stress. The fact that glycosyl hesperetin has a perspiration promoting effect is an unexpected physiological function of glycosyl hesperetin that has not been able to predict at all prior to the filing of this application.

### Effect of the Invention

The heat stress relieving agent of the present invention is a heat stress relieving agent containing glycosyl hesperetin as an effective ingredient, which can be orally ingested by humans on a daily basis continuously, safely, easily, and without discomfort, and can be provided at an industrially low cost. According to the heat stress relieving agent of the present invention, perspiration under a heat stress environment can be effectively promoted. Further, according to the heat stress relieving agent of the present invention, it is possible to effectively suppress an increase in the core body temperature under a heat stress environment. Especially, the heat stress relieving agent of the present invention has an excellent characteristic in which a desired effect is more remarkably exhibited when it is applied to a person who is concerned about heat illness or the like in a so-called heat stress environment such as working in the summer season or under blazing sun.

The basal body temperature maintaining agent of the present invention is a basal body temperature maintaining agent containing glycosyl hesperetin as an effective ingredient, which can be safely and easily taken orally by humans continuously on a daily basis without discomfort, and which can be provided at an industrially low cost. According to the basal body temperature maintaining agent of the present invention, it is possible to effectively maintain the basal body temperature. Especially, the basal body temperature maintaining agent of the present invention has an excellent feature that the intended action and effect are more remarkably exhibited when it is applied to a person who is likely to have a body temperature control disorder such as hypothermia, a chronic cold disorder, hot flashes, flashing, a hot flash due to menopause disorder or the like, not limited to the cooling caused by the so-called cold stress environment such as winter and cooling by air-conditioner.

### BRIEF DESCRIPTION OF THE DRAWINGS

[Figure 1]

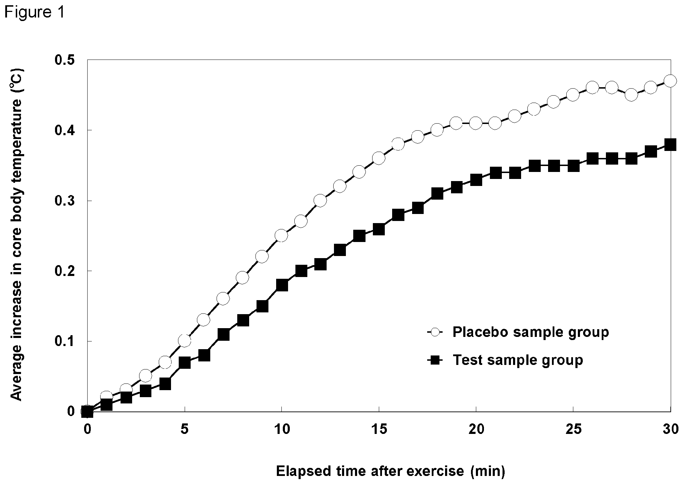

   Fig. 1 shows the change over time in the average increase in core body temperature of subjects under a heat stress environment (exercise load under room temperature of 30°C, 50% relative humidity) at a single ingestion of glycosyl hesperetin.
[Figure 2]

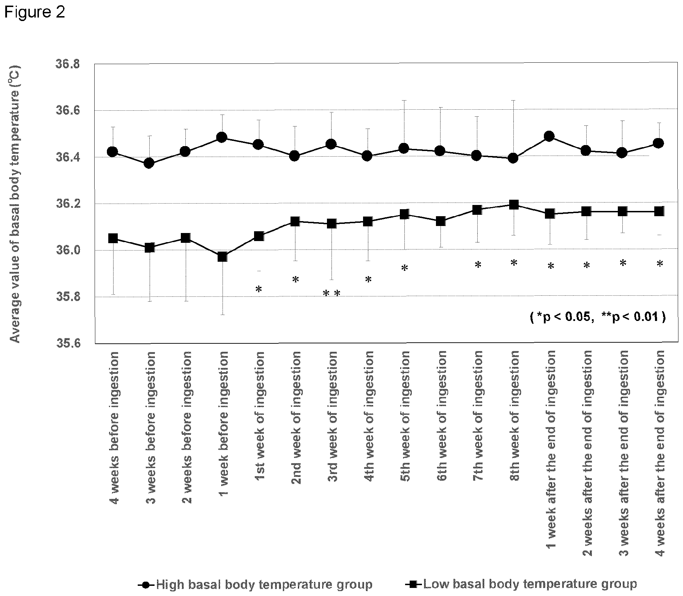

   Fig. 2 shows the change in basal body temperature of subjects during long-term ingestion of glycosyl hesperetin.

### MODE FOR CARRYING OUT THE INVENTION

The following is a description of the modes of implementation of the invention. These are only examples of preferred embodiments of implementing the invention, and the invention is not limited in any way to these embodiments of implementation.

The present invention relates to a heat stress relieving agent comprising glycosyl hesperetin as an effective ingredient.

The heat stress as used in the present application includes the stimulus given to the body by labor factors such as exercise and work, in addition to the stimulus given to the body by climatic factors such as summer season, mid-summer days, hot summer days, tropical nights and urban heat island phenomena, and by environmental factors such as high temperature and high humidity in bathroom, hot springs, saunas, factories, and workplaces. In addition, the term "under heat stress environment" in this application means being in a situation where the subject is exposed to heat stress caused by the aforementioned climatic, environmental and/or labor work factors.

The heat stress relieving as used in this application means the perspirating acceleration and the suppression of the rising core body temperature in a heat stress environment, and the heat stress relieving agent means an agent that has heat stress relieving effects.

Glycosyl hesperetin used as an effective ingredient of the heat stress relieving agent of the present invention includes a compound in which saccharides are bonded to hesperetin in general, and as suitable examples include hesperidin, a 7-O-β-glucosyl hesperetin, and a α-glycosyl hesperidin.

Hesperidin is a compound represented by the structural formula shown in Chemical Formula 1 below, which is also called vitamin P and has a structure in which rutinose composed of rhamnose and glucose is bound to hesperetin, and is a component contained in a large amount in peels of citrus fruits and the like.

Chemical Formula 1:

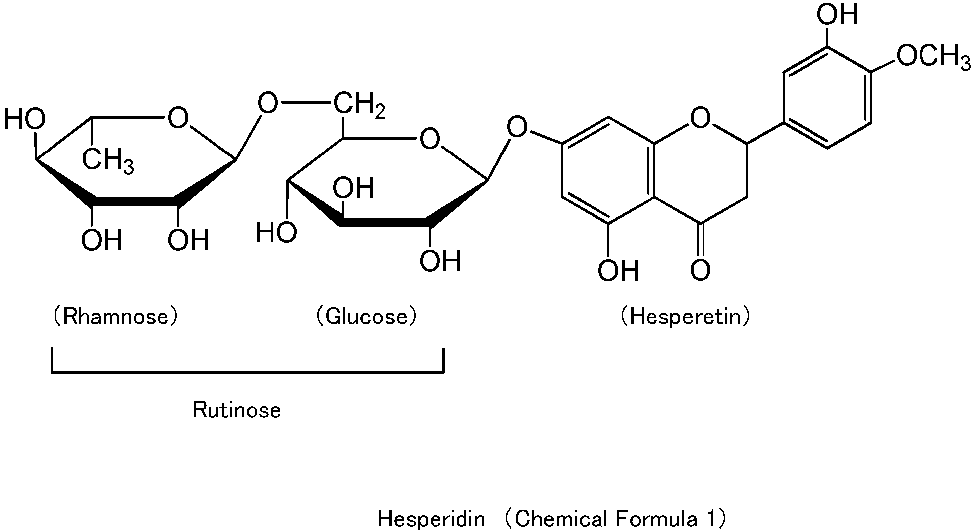

7-O-β-glucosyl hesperetin is a compound with a structure in which the rhamnose group that constitutes the rutinose of hesperidin is removed as shown in the following chemical formula 2, and which has characteristics of being more water-soluble than hesperidin. 7-O-β-glucosyl hesperetin can be produced, for example, by acting a α-L-rhamnosidase (EC 3.2.1.40) on a solution containing hesperidin as disclosed in JP-A H10-323196.

Chemical Formula 2:

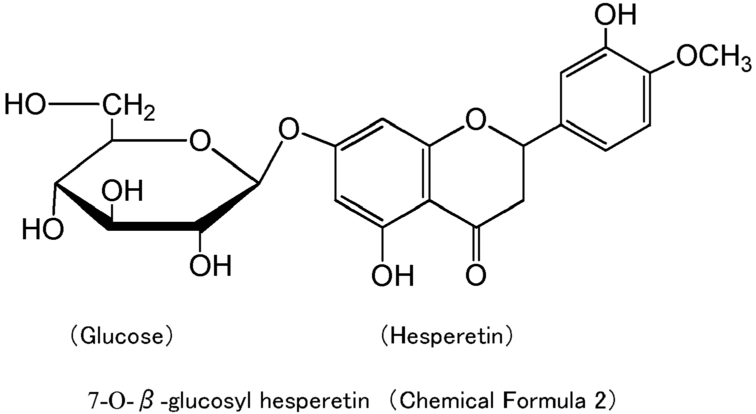

Further, α-glycosyl hesperidin is a compound in which an equimolar or more amount of saccharide (e.g., D-glucose, D-fructose or D-galactose) is α-linked to hesperidin. A representative example of α-glycosyl hesperidin is α-glucosyl hesperidin (also known as enzyme-treated hesperidin, glycosyltransfered hesperidin, water-soluble hesperidin, or glycosyltransfered vitamin P), as shown in the following chemical formula 3, in which one glucose molecule is α-linked to the glucose in the rutinose structure of hesperidin. For example, Hayashibara Hesperidin S (sold by Hayashibara Co., Ltd.) is commercially available as a product containing α-glucosyl hesperidin.

Chemical Formula 3:

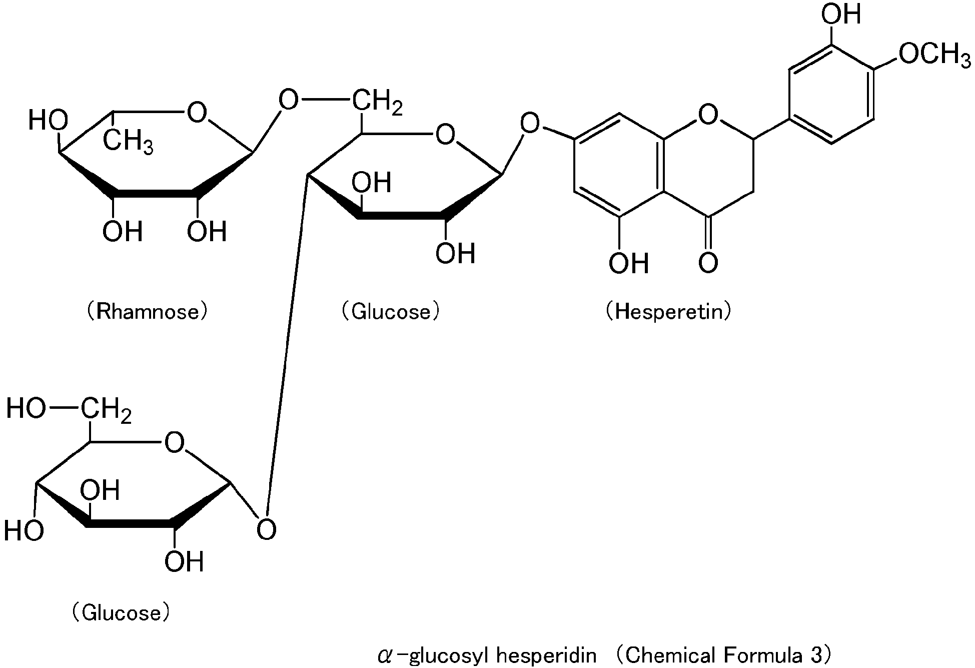

Glycosyl hesperetin is a highly safe and versatile substance that has been widely used as an ingredient in various food and beverage products, cosmetics, quasi-drugs, and pharmaceuticals. In particular, α-glycosyl hesperidin is more water-soluble than hesperidin and 7-O-β-glucosyl hesperetin, and which is easier to handle. The α-glycosyl hesperidin is hydrolyzed to hesperetin by the action of enzymes in vivo like hesperidin and 7-O-β-glucosyl hesperetin, and which exhibits the original biological activity of hesperetin.

In the present invention, glycosyl hesperetin can be used regardless of its origin, manufacturing method, purity and the like as long as it is glycosyl hesperidin, but a high-purity product containing less impurities is suitably used. As a high-purity product, glycosyl hesperetin obtained through a crystallization process may be used.

For example, the desired amount of hesperidin can be produced industrially by extracting hesperidin-containing plants, such as peels, seeds, and unripe fruits of citrus fruits and the like, using water, alcohol, or other solvents as appropriate. In addition, the desired amount of α-glycosyl hesperidin can be produced industrially at low cost by using a glycosyltransferase to produce α-glycosyl hesperidin in a solution containing hesperidin and α -glucosyl saccharide compounds, as disclosed in JP 11-346792. Furthermore, 7-O-β-glucosyl hesperetin is produced by allowing α-L-rhamnosidase to act on hesperidin and liberating rhamnose from hesperidin, and by collecting, the desired amount of 7-O-β-glucosyl hesperetin can be industrially manufactured at low cost.

In addition, the same applicant as the present application has established glycosyl hesperetin with reduced miscellaneous taste, coloration and odor (Hereinafter, "miscellaneous taste, coloration, and odor" may be collectively referred to as "miscellaneous taste and the like") and a method for producing the same by improving its production method with the aim of eliminating miscellaneous taste, coloration, and other defects of glycosyl hesperetin, which were previously considered to be defects of glycosyl hesperetin, and disclosed in the International Patent Publication WO2015/133483. Glycosyl hesperetin with reduced miscellaneous tastes and the like has the excellent advantage that humans can be ingested orally on a daily basis continuously, safely, easily and without discomfort, and can be provided industrially at low cost. Therefore, glycosyl hesperetin with reduced miscellaneous tastes and the like can be advantageously used as a glycosyl hesperetin suitable for the present invention.

Also, in the present application, when simply referring to glycosyl hesperetin, it may mean a glycosyl hesperetin mixture consisting mainly of one or more of hesperidin, α-glycosyl hesperidin, and 7-O-β-glucosyl hesperetin, unless otherwise specified. Further, glycosyl hesperetin used as an effective ingredient is preferably one containing a α-glycosyl hesperidin, and one containing a α-glucosyl hesperidin as a α-glycosyl hesperidin are preferred because the desired effect in the present invention, that is, the effect of promoting perspiration, suppressing the rising in core body temperature and the like can be more remarkably exhibited.

The glycosyl hesperetin content as used herein means the sum of the content of each component. It is determined by sampling the glycosyl hesperetin of the present invention; diluting or dissolving the sample with refined water to give a concentration of, for example, 0.1 w/w %; filtering the resulting solution with a commercialized 0.45 µm membrane filter; subjecting the filtrate to high-performance liquid chromatography (called "HPLC", hereinafter) using a reagent grade hesperidin, commercialized by Wako Pure Chemical Industries, Ltd., Tokyo, Japan, as a standard substance under the following conditions; and calculating the glycosyl hesperetin content based on the area of each peak appeared in the chromatogram at an UV wavelength of 280 nm and the molecular weight corresponding to each peak for each glycosyl hesperetin such as hesperidin, α-glycosyl hesperidin (α-glucosyl hesperidin, etc.), or 7-O-β-glycosyl hesperetin. Methods for quantifying the contents of the respective components corresponding to glycosyl hesperetin by HPLC are as shown in (1) to (4) below.

### <HPLC Analytical conditions>

HPLC Apparatus: "LC-20AD", commercialized by SHIMADZU CORPORATION
Degasser: "DGU-20A3", commercialized by SHIMADZU CORPORATION
Column: "CAPCELL PAK C18 UG 120", commercialized by Shiseido Co., Ltd
Detection: "SPD-20A", a UV detector, commercialized by SHIMADZU CORPORATION, Data processor: "CHROMATOPAC C-R7A" commercialized by SHIMADZU

### CORPORATION

Sample injection volume: 10 µL
Eluant: water/acetonitrile/acetic acid (80/20/0.01 (by volume ratio))
Flow rate: 0.7 ml/min
Temperature: 40°C
Measurement wavelength: 280 nm

### <Quantification of glycosyl hesperetin>

(1) Content of hesperidin: It is analyzed on HPLC and calculated based on the ratio of a peak area of hesperidin in a sample to that of a reagent grade hesperidin as a standard substance, commercialized by Wako Pure Chemical Industries, at a prescribed concentration.
(2) Content of α-glucosyl hesperidin: It is analyzed on HPLC and calculated based on the ratio of a peak area of α-glucosyl hesperidin in a sample to that of a reagent grade hesperidin as a standard substance, commercialized by Wako Pure Chemical Industries, at a prescribed concentration; and on the molecular weight ratio of α-glucosyl hesperetin to hesperidin.
(3) Content of 7-O-β-glucosyl hesperetin: It is analyzed on HPLC and calculated based on the ratio of the peak area of 7-O-β-glucosyl hesperetin in a sample to that of a reagent grade hesperidin as a standard substance, commercialized by Wako Pure Chemical Industries, at a prescribed concentration; and on the molecular weight ratio of 7-O-β-glucosyl hesperetin to hesperidin.
(4) Contents of other glycosyl hesperetins: They are analyzed on HPLC and calculated based on the ratio of a peak area of each of the other glycosyl hesperetins in a sample to that of a reagent grade hesperidin as a standard substance, commercialized by Wako Pure Chemical Industries, at a prescribed concentration; and on the ratio of the molecular weight of respective other glycosyl hesperetins to hesperidin.

As described above, the glycosyl hesperetin which is contained as an effective ingredient of the heat stress relieving agent of the present invention means a composition containing 1 or more kinds selected from (1) hesperidin, α-glycosyl hesperidin (a-glucosyl hesperidin, etc.), and 7-O-β-glucosyl hesperetin, which are the compounds having a hesperetin skeleton, as a main ingredient, that is, a glycosyl hesperetin mixture. In addition, the glycosyl hesperetin may contain compounds which are considered to be derived from the raw material of the production or to be generated as by products in the manufacturing process thereof, (2) flavonoids such as narirutin, diosmin, neoponcillin and glucosyl nariltin, and (3) trace components such as salts. The said glycosyl hesperetin may contain hesperetin to the extent that the desired effect of the invention is not interfered. The glycosyl hesperetin used in the present invention can also be blended with hesperetin whose dispersibility in water or other solvents has been enhanced by known physical or chemical methods or the like, to the extent that the desired effects of the present invention are not interfered.

The glycosyl hesperetin contained as the effective ingredient of the heat stress relieving agent of the present invention, that is, the glycosyl hesperetin mixture is usually, on a dry solid basis, at least 50% by mass but less than 100% by mass, preferably at least 60% by mass but less than 100% by mass, more preferably at least 70% by mass but less than 100% by mass, further preferably at least 80% by mass but less than 100% by mass, and more further preferably at least 85% by mass but less than 100% by mass. The upper limit of the glycosyl hesperetin contained as the effective ingredient of the heat stress relieving agent of the present invention is usually, on a dry solid basis, 99% by mass or lower which can be provided in relatively large quantities, affordably, and easily from an industrial viewpoint, and it may be 98% by mass or lower to be provide at a more lower cost; and it may be a low content of 97% by mass or lower to be provide at a further lower cost. However, in the case of the content on a dry solid basis, of glycosyl hesperetin in the glycosyl hesperetin of the present invention is relatively low, it should inevitably be required in a larger amount than in the case of using the one with a higher content of glycosyl hesperetin, resulting in a laborious and unfavorable handling. To avoid such defect, the lower limit of the glycosyl hesperetin content is usually at least, on a dry solid basis, 50% by mass, preferably at least 60% by mass, more preferably at least 70% by mass, and further preferably at least 80% by mass, more further preferably at least 85% by mass.

A more suitable form of glycosyl hesperetin to be included as an effective ingredient in the heat stress relieving agent of the present invention is α-glycosyl hesperidin, wherein said glycosyl hesperetin is a α-glycosyl hesperidin comprising a α-glucosyl hesperidin. In this case, a suitable content of the α-glucosyl hesperidin in glycosyl hesperetin is usually, on dry solid basis, 60% by mass or more and less than 100% by mass, preferably 70% by mass or more and less than 100% by mass, and more preferably 75% by mass or more and less than 100% by mass. The upper limit of the α-glucosyl hesperidin content in glycosyl hesperetin to be included as an effective ingredient in the heat stress relieving agent of the present invention is basically less than 100% by mass as described above. In order to provide the heat stress relieving agent of the present invention at a lower cost, the content may be as low as 99 % by mass, more preferably 95% by mass or less, and even more preferably 90% by mass or less, which can usually be provided industrially in relatively large quantities, inexpensively and easily. The lower limit of the α-glucosyl hesperidin content should usually be 60% by mass or more, preferably 70% by mass or more, and more preferably 75% by mass or more, for the same reason as the glycosyl hesperetin content in the glycosyl hesperetin mixture above.

The present invention relates to a basal body temperature maintaining agent comprising glycosyl hesperetin as an effective ingredient.

The average basal body temperature in humans is approximately 36.5°C, which is considered to be the temperature at which enzymes are most activated in the body, immune function is active, and health is maintained. However, in recent years, the basal body temperature has caused a decrease due to the following factors: for example, the effects of dietary habits such as not eating breakfast, unreasonable dieting, lack of minerals, vitamins, and proteins, and smoking; modulation of lifestyle rhythms such as a night-time lifestyle, sleep deficiency, and physical inactivity during daytime; surrounding environment such as winter season and cooling; ageing; and the like. The body temperature of humans whose basal body temperature has decreased in this way is known 0.5 to 1.5°C lower than the average basal body temperature. Such a decrease in basal body temperature causes discomfort such as chills and fatigue, delayed wound healing, lowered immunity, lowered metabolism, lowered enzyme activities in the body, lowered blood pressure, poor circulation such as coldness, stiff shoulders, back pain, and joint pain, deterioration of internal organ functions such as constipation and diarrhea, nervous system disorders such as depression, and disturbances in physical functions, and can lead to death due to extreme decrease in basal body temperature. Therefore, proper regulation of basal body temperature to keep it close to 36.5°C, the average basal body temperature, is important for maintaining good health.

The basal body temperature referred to as in this application means the body temperature measured in a resting state at the time of awakening, and generally measured under the tongue. The basal body temperature maintenance in this application means that the basal body temperature is kept close to the average basal body temperature of 36.5°C. A person who tends to have a low basal body temperature as used in this application, means a person whose average basal body temperature under the tongue is less than 36.2°C, or strictly less than 36.1°C, further strictly less than 36.0°C. A person who tends to have a high basal body temperature as used in this application, means a person whose average basal body temperature under the tongue is 36.2°C or more, or strictly 36.3°C or more, further strictly 36.4°C or more.

Glycosyl hesperetin used as an effective ingredient of the heat stress relieving agent of the present invention described above, can also be used as an effective ingredient of a basal body temperature maintaining agent.

The heat stress relieving agent and the basal body temperature maintaining agent of the present invention comprise various glycosyl hesperetin as effective ingredients. Although the details will be described later, the effect of perspiration acceleration, the effect of suppressing rise in the core body temperature and the effect of maintaining basal body temperature can be exerted effectively by daily and continuous oral intake of the heat stress relieving agent and the basal body temperature maintaining agent that contain glycosyl hesperetin as an effective ingredient. The heat stress relieving agents and the basal body temperature maintaining agents containing glycosyl hesperetin as an effective ingredient are expected to prevent, alleviate and improve symptoms and diseases caused by heat stress, such as heat illness. In addition, the heat stress relieving agents and the basal body temperature maintaining agents containing glycosyl hesperetin as an effective ingredient are also expected to prevent, alleviate and improve skin disorders and skin quality such as swelling, dry skin, and detoxify the body; symptoms associated with sleep disorders and sleep disorders such as insomnia, difficulty of falling asleep, difficulty of staying asleep and difficulty of sleeping soundly; vasomotor nerve symptoms such as hot flashes, abnormal sweating, palpitations and dizziness associated with menopausal disorders and other menopausal disorders in male and female; neuropsychiatric symptoms such as emotional anxiety, tinnitus, irritability, anxiety, depression, insomnia, lightheadedness, lethargy, memory loss, malaise and fatigue; musculoskeletal symptoms such as stiff shoulders, lower back pain and joint pain; gastrointestinal symptoms such as nausea and loss of appetite; mucosal symptoms such as dry and itchy skin; genitourinary symptoms such as dysuria, frequent urination, sexual intercourse, and vulvar discomfort; lifestyle-related diseases and metabolic syndrome, such as dysglycemia, dyslipidemia, dysuricemia, and obesity; various diseases such as cardiac, hepatic, renal, brain, neurological, allergic, infectious, and malignant tumors. Therefore, heat stress relievers and basal body temperature maintainers that contain glycosyl hesperetin as an effective ingredient can be advantageously used as functional foods, health foods, beauty foods, nutritionally functional foods, health function foods, foods with function claims, foods for specified health uses, quasi-drugs, and pharmaceuticals for the prevention, alleviation, and improvement of the above-mentioned symptoms and diseases. Further, the heat stress relieving agent and the basal body temperature maintaining agent containing glycosyl hesperetin as an effective ingredient can also be used in other functional foods, health foods, beauty foods, nutritionally functional foods, health function foods, foods with function claims, specified health foods, quasi-drugs, pharmaceuticals, or foods and beverages.

In addition, when glycosyl hesperetin is used for the aforementioned purpose, and when it is used in combination with one or more ingredients such as other plant and animal-derived substances, extracts or compounds that have the effects of promoting perspiration, suppressing rise in core body temperature and maintaining basal body temperature, perspiration promoting, rise in core body temperature-suppressing, and basal body temperature-maintaining effects of glycosyl hesperetin are more effectively exerted, as well as the prevention, mitigation, and improvement of heat stress, skin disorders and skin quality, sleep disorders and symptoms associated with sleep disorders, menopausal disorders and symptoms associated with menopausal disorders, and the prevention, mitigation, and improvement of various diseases.

Other animal- and plant-derived substances, extracts, or compounds that have perspiration-promoting, rise in core body temperature-suppressing, and basal body temperature-maintaining effects in combination with glycosyl hesperetin are the following; for example, animal substances such as Bovine gallstones, Shinju, Borei, Mamushi, Earthworm epidermis, Yutan, Reiyoukaku, Rokujo and their extracts; plants or extracts thereof such as Artichoke, Eyebright, Aodamo, Aomizu, Akaza, Akane, Akayaziou, Akigumi, Agrimony, Agni fruit, Akebi, Asai fruit, Asatsuki, Assafoetida, Ashwagandha, Asunaro, Asenyakunoki, Ahi Amarillo, Ahi Limon, Rape, Abemaki, Jiaogulan, Anise, American witch hazel, Arisuchin, Arnica, Angelica, Barrenwort, Japanese knotweed, Italian parsley, Ginkgo, Ichiyakuso, Strawberry, Itohayuri, Itofunori, Catnip, Inubiwa, Inondo, Ipe bark, Irohamomiji, Iwatoyuri, Neem, Uikyo, Witch hazel, Winter Green, Turmeric, Marsh mallow, Udo, Ume, Urajirogashi, Ulupica, Epimedium brevicornum, Eijitsu, Echinacea, Egoma, Shallot, Siberian ginseng, Enishida, Hercampure, Elderberry, Erva mate, Oats, Allspice, Ougi, Palmyra palm, Oubaku, Ouren, Oazami, Otsuzurafuji, Oobagekitsu, Oobako, Obatanetsukebana, Barley, Okahijiki, Otaneninjin, Otomeyuri, Onita, Oniyuri, Oregano, Orange, Kava, Kakiodoshi, Kakikazura, Kahokuzansho, Pumpkin seed, Katsuaba bark, Kanokosou, Kanokoyuri, Turnip, Kabosu, Chamomile, Karashina, Karatachi, Guarana, Roselle, Cardamom, Curry plant, Wild gingers, Licorice, Kanto, Kikyo, Gishigishi, Kidachitogarashi, Caraway, Gyojaninniku, Apricot kernel, Kumquat, Calendula, Harlequin, Kamala, Kudzu, Kuchinashi, Cumin, Kurara root, Clary sage, Wheel lily, Walnut, Grapefruit, Watercress, Clove, Black mustard, Keigai, Caper, Cassia seed, Gennoshoko, Kokemomo, Kosho, Konagi, Coriander, Sagarame, Pomegranate, Sweet potato, Sanebutonatsume, Saffron, Sarsaparilla, Hawthorn, Sansho, Shikuwasa, Shishiudo, Shishitogarashi, Perilla, Winged bean, Shikon, Shinanikei bark/tree branch / root bark, Shimotsukeso, Chinese peony, Jasmine, Shakuyaku, Mondo grass, Ginger, Shoma, Shouyoudaiou, Shirayamagiku, White mustard, White goosefoot, Shiroyomena, Agarwood, Sudachi, Stevia, Supeinkanzou, Sage, Savory, Stinging nettle, Plantain, St. John's wort, Valerian, Mustard greens, Dandelion, Elder, Butterbur, Horseradish, Ceylon cinnamon bark / tree branch / root bark, Sekisetsusou, Seri, Senkyu, Senburi, Buckwheat, Soreru, Daiou, Daikon, Oswego tea, Thyme, Takasagoyuri, Tade, Tanetsukebana, Dabira rugosa, Onion, Tamotoyuri, Tarragon, Chicory, Chervil, Chives, Chadebugure, Chouji, Tinnevelly senna, Tsubokusa, Tree onion, Chinese knotweed, Daisy, Dill, Devil's claw, Tsuwabuki, Easter lily, Devil's claw root, Tencha, Chili pepper, Touki root, Star anise fruit, Tounin, Dokudami, Passion flower, Togenashi, Tochibaninjin, Tochu leaf, Tomato, Tormentilla, Nagabagishigishi, Feverfew, Jujube, Worm wood, Nira, Niwatoko, Garlic, Nouzenharen, Saw palmetto, Nokongiku, Nobiru, Welsh onion, Basil, Mint, Hakatayuri, Bush clover, Hagoromogusa, Hajikami, Hasuimo, Parsley, Patchouli, Passion flower, Passion fruit, Hatomugi, Vanilla, Habanero, Paffia root, Sweet pepper, Hamasuge, Hamabishi, Jack fruit, Jalapeno, Bell pepper, Hikanzakura, Hyssop, Hinoki, Hihatsu, Hihatsumodoki, Sunflower, Himeuwabamisou, Himenira, Himeyuri, Birako, Piripiri, Betel Palm, Bhut jolokia, Futoukazura, Fenugreek seed, Fennel, Fuki, Fujimame, Black cohosh, Buntan, Luffa, Safflower, Benibanaborogiku, Henna, Rue, Boufu, Peony, Hop, Ephedra, Marjoram, Matatabi, Machilus, Pine, Matebashii, Mate Tea, Madonna Lily, Milk thistle seed, Horse chestnut seed, Rutaceae, Mishimasaiko root, Mitsuba, Mitsubautsugi, Myoga, Mube, Echinacea, Mozuku, Willow, Yabukanzou, Yamatogebanreishi, Japanese yam, Golden-rayed lily, Yamarakkyou, Yuzu, Yousai, Yomena, Lychee seed, Lime, Rakkyo, Lovage, Lavender, Lamb's-ear, Regal lily, Leek, Ryukyuchiku, Ryukyubaraichigo, Apple unripe fruit, Linden flower, Rooibos, Rocket, Rhubarb, Borage, Lettuce, Lemon, Lemon grass, Lemon thyme, Lemon verbena, Lemon balm, Lemon myrtle, Forsythia, Rose hip, Rose pink bud, Rosemary, Rose red, Roselle, Laurel, Rocoto, Rosea, Logwood, Wakegi, Wasabi, Burnet, Chinese milk vetch, Persimmon leaf, Licorice leaf, Black soybean testa, Black rice seed, Shell ginger leaf, Hosobayuri, Seiyouyanagi, and Chomeisou; extracts of seaweeds such as Hanafunori, Fukurofunori, and mafunori; extracts such as raw coffee bean, sweet potato shochu lees, polyporaceae mycelium and agaricus mycelium; polyphenols such as resveratrol, quercetin, chlorogenic acid, anthocyanins, curcumin, kaempferol, catechins, flavonoids; carotenoids such as astaxanthin, β-cryptoxanthin, lycopene, fucoxanthin, lutein, crocetin, retinol, retinal, retinoic acid, β-carotene; pyrocarpines such as pyrocarpine hydrochloride; ephedrine, aspirin, sodium salicylate, insulin, adrenaline, acetylcholine, salicin, salicylic acid, choline salicylate, diflunisal, etenzamid, mephenamic acid, diclofenac, slindac, indomethacin, fervinac, etodrac, tolmethine, naphthomen, ibprofen, fluruben, naproxene, phenobrophene, oxabrozine, loxoprofen, saltoprofen, ketrolac, pyroxycam, ampyroxycam, meroxycam, lornoxicum, tenoxycam, selecoxib, valdecoxyb, parecoxyb sodium, thiaramid hydrochloride, tynolysine hydrochloride, epilyzole, emorphazone, mepyrizol, amino phenylbutazone, oxyphenylbutazone, ketophenylbutazone, feprazone, sulfinpyrazone, antipyrine, sulpyrine, isopropylantipyrine, glycine, theanine, GABA, sake yeast, diphenhydramine hydrochloride, solpidem tartrate, triazolam, zopiclone, eszopiclone , etizolam, brothizolam, lilmazafone hydrochloride, lolmetazepam, flunitrazepam, estazolam, nitrazepam, quazepam, flurazepam hydrochloride, haroxazolam, lamerteon, subolexant, miltazapin, amitriptyrin, myanthazapine, amitriptyrin, capsaicin, dihydrocapsaicin, ginger, gingerol, gingerol, gingerone, gingeriberen, piperin, sanshool, sanshamide, gellanol, allyl isothiocyanate, phenethylisothiocyanate, sinigrin, sinarbin, sulforaphan glucosinolate, equol, isoflavone, L-tyrosine, L-Phenylalanine, L-Tryptophan, 1,3,5-trihydroxybenzene, 3,4-dihydroxyphenylacetic acid, L-3,4-dihydroxyphenylalanine, 4- (2-aminoethyl) benzene-1,2-Diol, noradrenaline, adrenaline, 5-hydroxytryptamine, caffeic acid, ifenprodil, α-lipoic acid, 4-hydroxychalcone, ergothioneine, resveratrol, carnosin, carnitine, salicylic acid hydrobromide, cinnapic acid, fel Rolic acid, silicic acid, tocopheryl nicotinate, α-tocopherol, β-tocopherol, γ-tocopherol, δ-tocopherol, α-tocotrienol, β-tocotrienol, γ-tocotrienol, δ-tocotrienol, nicotinamide, proanthocyanin; chlorogenic acids such as hydrolyzable tannins, catechol, chlorogenic acid, isochlorogenic acid, neochlorogenic acid, cryptochlorogenic acid, caffe oil quinic acid; leucocyanidin, prunin, procyanidol oligomer, glucosyl rutin, glucosyl naringin.

The intake of the heat stress relieving agent and basal body temperature maintaining agent of the present invention can be adjusted according to the frequency of intake of the agent and the body condition of the person who takes the agent. When all glycosyl hesperetin as effective ingredients, are considered to be hesperidin and converted to mass (hereinafter in this application, simply referred to as "hesperidin equivalent"), the number of ingestion of glycosyl hesperetin is usually 1 to 5 times a day, preferably 2 to 3 times a day, and the dosage of hesperidin equivalent is 10 to 3,000 mg, preferably 20 to 2,000 mg, more preferably 30 to 1,000 mg, and further more preferably 50 to 500 mg per one ingestion, respectively. Further, the ingestion period of the heat stress relieving agent and the basal body temperature maintaining agent of the present invention is such that the desired effect can be obtained even if the ingestion is once or several times, but it is desirable to take it for a week or more, preferably 2 weeks or more, more preferably 4 or more and further more preferably for 8 weeks or more. When the intake is below the lower limit, the desired effect may be significantly reduced or may not be exerted, and when the intake is above the upper limit, the effect will not be commensurate with the dosage, which is not desirable from the viewpoint of economic efficiency.

The heat stress relievers and basal body temperature maintaining agents of the present invention can be used as various solid, granulated, powdered, suspended, paste, jelly, and liquid forms, such as lozenges, liver oil drops, complex vitamins, mouth fresheners, mouth fragrant tablets, oral and tube feedings, and oral medications, and can be optionally formulated into various compositions in the aforementioned forms. As for the methods to use as the above forms and the method to be contained in various compositions in the above forms, one or more known methods such as mixing, kneading, blending, adding, dissolving, dipping, penetrating, spreading, coating, spraying, and injecting, can be used as appropriate in the process until those compositions are completed. The amount of glycosyl hesperetin to be contained as an effective ingredient in the heat stress relieving agent and basal body temperature maintaining agent of the present invention is optional as long as the desired effect can be achieved.

The present invention is explained in more detail by the following experiments.

### <Experiment 1: Effect of glycosyl hesperetin intake on perspiration amount and core body temperature of subjects under heat stress>

A test sample containing glycosyl hesperetin or a placebo sample was ingested by subjects, and the test was conducted to measure changes in perspiration amount and core body temperature in a heat stress environment (exercise load under room temperature of 30°C, 50% relative humidity).

### <Experiment 1-1: Preparation of glycosyl hesperetin>

Glycosyl hesperetin was prepared according to the method described in Example 1 of the International Patent Publication WO2015/133483. That is, four parts by mass of 1 N aqueous sodium hydroxide solution was heated to 80°C and added with one part by mass of hesperidin and seven parts by mass of dextrin (dextrose equivalent (DE) of 20), followed by dissolving the contents by stirring for 30 min, adjusting the solution to pH 9.0, then added 30 units/g-dextrin of cyclodextrin glucanotransferase, derived from *Geobacillus stearothermophilus* Tc-91 strain (deposit number: FERM BP-11273, National Institute of Technology and Evaluation, NITE Patent Microorganisms Depositary (NPMD), 2-5-8 Kazusakamatari, Kisarazu City, Chiba, Japan) and subjected to an enzymatic reaction for 18 hours while keeping the pH at 6.9 and the temperature at 50°C. After inactivating the remaining enzyme in the resulting enzymatic reaction solution, "GLUCOZYME", a product name of a glucoamylase, commercialized by Nagase ChemteX Corporation was added in an amount of 100 units per gram solids of the enzymatic reaction solution, and enzymatic reaction was carried out for five hours while keeping the pH at 5.0 and the temperature at 55°C to form α-glucosyl hesperidin. The resulting enzymatic reaction solution was heated to inactivate the remaining enzyme and filtered, followed by feeding the resulting filtrate to a column packed with a porous synthetic adsorbent, product name "DIAION HP-10" commercialized by Mitsubishi Chemical Corporation, at a space velocity (SV) of two. Thereafter, the column was fed with refined water for washing and further fed with an aqueous ethanol solution while increasing stepwisely the concentration of ethanol to collect fractions containing α-glucosyl hesperidin, and concentrating the pooled fractions *in vacuo,* and pulverizing the concentrate to obtain a pale yellow particulate glycosyl hesperetin. The resulting particulate glycosyl hesperetin contained 80.0% by mass of α-glucosyl hesperidin, 12.3% by mass of hesperidin, and 7.7% by mass of other ingredients, and was equivalent to a commercially available glycosyl hesperetin (product name "Hayashibara Hesperidin S", sold by Hayashibara Co., Ltd.).

### <Experiment 1-2: Preparation of test sample>

As shown in Table 1, the test sample was prepared by completely dissolving 250 mg of the glycosyl hesperetin powder obtained in Experiment 1-1 in 200 mL of commercially available near-water (tangerine flavor). As a placebo sample, said commercially available near-water was used.

**Table 1**

| | Test sample | Placebo sample |
|---|---|---|
| Commercially available near-water (tangerine flavor) | 200 mL | 200 mL |
| Glycosyl hesperetin prepared in Experiment 1-1 | 250 mg | |

### <Glycosyl hesperetin Ingestion Test>

As the subjects, four healthy adult males (26 to 51 years old), were made to take 200 mL of the sample without informing them whether the sample to be taken was the test sample or the placebo sample. The subjects were allowed to rest for 20 minutes after ingestion, and then exercised in a room controlled at 30°C room temperature and 50% relative humidity. As the exercise load, the subjects were made to execute a step-up and down exercise with a height of 15cm stepstool, 20 times per minute for 30 minutes. During the test, all subjects were dressed in commercially available clothing of the same material. The test was performed a double-blind, crossover, single-dose test. That is, subjects who were given the test sample first were given the placebo sample later, and subjects who were given the placebo sample first were given the test sample later, the test was performed in the same manner. The test was performed one day apart to eliminate the influence of the previously ingested sample.

### <Evaluation items and methods>

The evaluation items were the amounts of perspiration and changes in core body temperature under the heat stress environment (exercise load under room temperature of 30°C, 50% relative humidity). The perspiration amounts were determined by subtracting the weight of the clothing before the exercise load from the weight of the subject's clothing after the exercise load, and the average of the four subjects' perspiration amounts due to the exercise load was calculated. The core body temperature was measured every minute from the start of the exercise load to the end of the exercise load (30 minutes later). The core body temperature during the exercise load was measured with a data collecting handy thermometer ("LT-8A", Gram Corporation) by using an ear-type body temperature measurement probe utilizing a thermistor temperature sensor. The average increase in core body temperature was calculated by taking the core body temperature immediately before the exercise load as the reference value and determining the amount of temperature change every minute from the start of the exercise load, and then calculating the average value of four subjects. The average perspiration amount of the subjects in the heat stress environment is shown in Table 2. The average change in core body temperature in the heat stress environment is shown in Figure 1 and Table 3.

**Table 2**

| | Average perspiration amount (g) |
|---|---|
| Placebo sample ingestion group | 55.2 ± 32.7 |
| Test sample ingestion group | 59.4 ± 34.2 |

As shown in Table 2, the average perspiration amount of the subjects in this test was 55.2 ± 32.7g in the placebo sample ingestion group compared with 59.4 ± 34.2g in the test sample ingestion group, which clearly showed high value. That is, it was confirmed that the perspiration amount of the subjects under the heat stress environment was remarkably increased by the ingestion of glycosyl hesperetin.

**Table 3**

| Elapsed time after exercise (min) | Average increase in core body temperature (°C) | |
|---|---|---|
| | Placebo sample ingestion group | Test sample ingestion group |
| 0 | 0.00 ± 0.00 | 0.00 ± 0.00 |
| 1 | 0.02 ± 0.01 | 0.01 ± 0.01 |
| 2 | 0.03 ± 0.01 | 0.02 ± 0.02 |
| 3 | 0.05 ± 0.02 | 0.03 ± 0.05 |
| 4 | 0.07 ± 0.02 | 0.04 ± 0.06 |
| 5 | 0.10 ±0.03 | 0.07 ± 0.07 |
| 6 | 0.13 ±0.03 | 0.08 ± 0.07 |
| 7 | 0.16 ± 0.04 | 0.11 ± 0.08 |
| 8 | 0.19 ±0.05 | 0.13 ±0.09 |
| 9 | 0.22 ± 0.05 | 0.15 ± 0.09 |
| 10 | 0.25 ± 0.06 | 0.18 ± 0.09 |
| 11 | 0.27 ± 0.07 | 0.20 ± 0.09 |
| 12 | 0.30 ±0.07 | 0.21 ± 0.10 |
| 13 | 0.32 ± 0.07 | 0.23 ± 0.11 |
| 14 | 0.34 ± 0.08 | 0.25 ± 0.11 |
| 15 | 0.36 ± 0.08 | 0.26 ± 0.11 |
| 16 | 0.38 ± 0.09 | 0.28 ± 0.11 |
| 17 | 0.39 ±0.10 | 0.29 ± 0.11 |
| 18 | 0.40 ± 0.10 | 0.31 ± 0.11 |
| 19 | 0.41 ± 0.10 | 0.32 ± 0.10 |
| 20 | 0.41 ± 0.10 | 0.33 ± 0.10 |
| 21 | 0.41 ± 0.10 | 0.34 ± 0.10 |
| 22 | 0.42 ± 0.10 | 0.34 ± 0.10 |
| 23 | 0.43 ± 0.10 | 0.35 ± 0.10 |
| 24 | 0.44 ± 0.09 | 0.35 ± 0.10 |
| 25 | 0.45 ± 0.10 | 0.35 ± 0.11 |
| 26 | 0.46 ± 0.10 | 0.36 ± 0.12 |
| 27 | 0.46 ±0.11 | 0.36 ± 0.12 |
| 28 | 0.45 ±0.11 | 0.36 ± 0.13 |
| 29 | 0.46 ±0.11 | 0.37 ± 0.14 |
| 30 | 0.47 ± 0.12 | 0.38 ± 0.12 |

As also shown in Table 3, in the placebo sample ingestion group, the average increase in core body temperature was 0.10 ± 0.03°C at 5 minutes after the start of exercise load, 0.25 ± 0.06°C at 10 minutes, 0.41 ± 0.10°C at 20 minutes, and 0.47 ± 0.12°C at 30 minutes, and increased with time. On the other hand, in the test sample ingestion group, the average increase in core body temperature was 0.07 ± 0.07°C at 5 minutes, 0.18 ± 0.09°C at 10 minutes, 0.33 ± 0.10°C at 20 minutes, and 0.38 ± 0.12°C at 30 minutes after the start of exercise. Further, as shown in Figure 1, the average increase in core body temperature in the test sample ingestion group (symbol "■" in Figure 1) was clearly more gradual than that in the placebo sample ingestion group (symbol "o" in Figure 1). Therefore, it was shown that the ingestion of the test sample containing glycosyl hesperetin significantly suppressed the increase in the core body temperature under the heat stress environment.

As shown in Tables 2, 3, and Figure 1, it was confirmed that in the heat stress environment, glycosyl hesperetin ingestion increased perspiration amount and suppressed the rising core body temperature, and therefore, glycosyl hesperetin is found to be useful as an effective ingredient of heat stress relieving agent.

### <Experiment 2: Effect of long-term ingestion of glycosyl hesperetin on basal body temperature>

The test sample in the form of a tablet (740 mg / tablet) having the composition shown in Table 4 below was ingested by the subject for a long-term, and the change in basal body temperature was measured.

**Table 4**

| Ingredient name | Content (% by mass) |
|---|---|
| Glycosyl hesperetin prepared in Experiment 1-1 | 35.000 |
| Maltose | 35.427 |
| Erythritol | 16.000 |
| Pullulan | 0.080 |
| Yuzu juice powder | 10.000 |
| Flavoring | 1.000 |
| Acidulant | 1.000 |
| Sucrose fatty acid ester | 1.200 |
| Sweetener (aspartame / L-phenylalanine compound) | 0.200 |
| Sweetener (sucralose) | 0.058 |
| Sweetener (acesulfame potassium) | 0.035 |

### <Long-term ingestion study of glycosyl hesperetin>

Eighteen healthy adult males (23 to 63 years old), were set as subjects for the test. The test sample (a tablet having glucosyl hesperetin with the composition shown in Table 4 above) was ingested by the subjects once a day at any given time for 8 consecutive weeks.

### <Evaluation items and methods>

The basal body temperature of subjects was measured and recorded by subjects themselves using a basal body thermometer (model number "C531", Terumo Corporation) in a resting state every morning upon waking. In detail, the basal body temperature of subjects was measured by placing the tip of the basal body thermometer under the tongue (back side of the tongue) and holding with the mouth lightly closed for 5 minutes. The basal body temperature measurements were taken for a total of 16 weeks, starting 4 weeks before the test sample ingestion as a pre-test observation, followed by 8 weeks of the test sample ingestion, and then ending 4 weeks after the end of the test sample ingestion. After that, the average and standard deviation of the basal body temperature for each week were calculated.

The results obtained were divided into following two groups based on the average value of the basal body temperature at the timing of one week before the sample ingestion, and stratified analysis was performed; the high basal body temperature group (9 subjects whose basal body temperatures tended to be high, with an average value of basal body temperature of each subject was 36.2°C or higher during one week before sample ingestion) and the low basal body temperature group (9 subjects whose basal body temperatures tended to be low, with an average value of basal body temperature of each subject was less than 36.2°C during one week before sample ingestion). The results of the stratified analysis are shown in Table 5 and Figure 2.

### <Statistical analysis>

Measurement data were expressed as means ± standard deviation (SD). For statistical analysis, Microsoft Excel 2013 (Microsoft Japan Co., Ltd.) was used to compare the mean values with those of one week before the test, and statistical processing was performed using paired t-test. A significance level (p-value) of less than 0.05 (risk rate less than 5%) was considered to be significant.

**Table 5**

| Measurement period | Average value of basal body temperature (°C) | |
|---|---|---|
| | High basal body temperature group | Low basal body temperature group |
| 4 weeks before ingestion | 36.42 ± 0.11 | 36.05 ± 0.24 |
| 3 weeks before ingestion | 36.37 ± 0.12 | 36.01 ± 0.23 |
| 2 weeks before ingestion | 36.42 ± 0.10 | 36.05 ± 0.27 |
| 1 week before ingestion | 36.48 ± 0.10 | 35.97 ± 0.25 |
| 1st week of ingestion | 36.45 ± 0.11 | 36.06 ± 0.15 * |
| 2nd week of ingestion | 36.40 ± 0.13 | 36.12 ± 0.17 * |
| 3rd week of ingestion | 36.45 ± 0.14 | 36.11 ± 0.24 ** |
| 4th week of ingestion | 36.40 ± 0.13 | 36.12 ± 0.17 * |
| 5th week of ingestion | 36.43 ± 0.21 | 36.15 ± 0.15 * |
| 6th week of ingestion | 36.42 ± 0.19 | 36.12 ± 0.11 |
| 7th week of ingestion | 36.40 ± 0.17 | 36.17 ± 0.14 * |
| 8th week of ingestion | 36.39 ± 0.25 | 36.19 ± 0.13 * |
| 1 week after the end of ingestion | 36.48 ± 0.12 | 36.15 ± 0.13 * |
| 2 weeks after the end of ingestion | 36.42 ± 0.11 | 36.16 ± 0.12 * |
| 3 weeks after the end of ingestion | 36.41 ± 0.14 | 36.16 ± 0.09 * |
| 4 weeks after the end of ingestion | 36.45 ± 0.09 | 36.16 ± 0.10 * |

| | | |
|---|---|---|
| (*p<0.05, **p<0.01) | | |

As shown in Table 5 and Fig. 2, for subjects who ingested the test sample containing glycosyl hesperetin, the average values of basal body temperature at each week from 4 week before ingestion to 8 weeks and after starting ingestion to 4 weeks after ingestion was not significantly different from the average values of basal body temperature at 1 week before ingestion of the test sample in the high basal body temperature group. In contrast, the average value at 1 to 5 weeks after ingestion, 7 to 8 weeks after ingestion of the test sample and at 1 to 4 weeks after ingestion of the test sample was significantly higher than the average values of basal body temperature at 1 week before ingestion of the test sample in the low basal body temperature group. (significant level (p value) was less than 0.05, and significant level (p value) was less than 0.01 at 3 weeks after ingestion). That is, it was shown that in the high basal body temperature group (a group of subjects with a tendency to have a high basal body temperature of more than 36.2°C for one week before taking the test sample), the basal body temperature was maintained as it was at around 36.5°C, which is the average basal body temperature of humans, after the ingestion of the test sample containing glycosyl hesperetin. While it was shown that in the low basal body temperature group (a group of subjects with a tendency to have a low basal body temperature of less than 36.2°C for one week before taking the test sample), the basal body temperature increased and approached the proper basal body temperature zone around 36.5°C, which is the average basal body temperature of humans, after the ingestion of the test sample containing glycosyl hesperetin.

As clearly shown in Figure 2, when the test sample containing glycosyl hesperetin was ingested, it was confirmed that the average basal body temperature of the high basal body temperature group (symbol "•" in Figure 2) was maintained at around 36.5°C which is the average basal body temperature of humans, while the average basal body temperature of the low basal body temperature group (symbol "■" in Figure 2) was increased to bring it closer to the appropriate temperature range around 36.5°C which is the average basal body temperature of humans. This result indicates that glycosyl hesperetin is useful as an effective ingredient in the basal body temperature maintaining agent.

Hereinafter, the present invention will be specifically described based on examples, but the present invention is not limited to thereto.

### Example 1

### <Heat stress relieving agent in powder form>

A commercially available α-glucosyl hesperidin-containing powder (product name "Hayashibara Hesperidin S", sold by Hayashibara Co., Ltd.) was used as a heat stress relieving agent. This product is a heat stress relieving agent which exhibits perspiration accelerating effect or suppressing effect on rise in core body temperature, with excellent storage stability and heat stability that can be added to various beverages such as water, tea, black tea, and coffee and the like and other food and beverage products to be ingested on a daily basis.

### Example 2

### <Heat stress relieving agent in tablet form>

One part by mass of glycosyl hesperetin-containing powder (a-glucosyl hesperidin 82% by mass, hesperidin 1% by mass, 7-O-β-glucosyl hesperetin 8% by mass, other components 9% by mass) (prepared by Hayashibara Co., Ltd.), 19 parts by mass of α, α-trehalose dihydrate crystal-containing powder (product name "TREHA", sold by Hayashibara Co., Ltd.), 0.1 parts by mass of citric acid, and 0.1 parts by mass of sodium chloride were uniformly mixed and tableted according to the conventional method to obtain a heat stress relieving agent in the form of a tablet of 250 mg. This product has an excellent perspiration accelerating effect or suppressing effect on rise in core body temperature, can alleviate or ameliorate the symptoms and can effectively delay or prevent the onset of heat illness in patients who are suffering from heat illness or a person who is concerned about the onset of heat illness to be usually taken daily by adults at a dose of 25 to 1,000 mg per day as the mass of above-mentioned glycosyl hesperidin.

### Example 3

### <Heat stress relieving agent in soft drink form>

In 5,000 parts by mass of water, 200 parts by mass of sugar, 100 parts by mass of α,α-trehalose dihydrate crystal-containing powder (product name "TREHA", sold by Hayashibara Co., Ltd.), 6 parts by mass of citric acid, 6 parts by mass of sodium chloride, 0.2 parts by mass of potassium chloride, 0.05 parts by mass of calcium lactate, 0.01 parts by mass of magnesium chloride, 5 parts by mass of α-glucosyl hesperidin crystal-containing powder (more than 99 % by mass pure, prepared by Hayashibara Co., Ltd.) and 0.1 parts by mass of ascorbic acid 2-glucoside anhydrous crystal-containing powder (product name "Ascofresh", sold by Hayashibara Co., Ltd.) were uniformly dissolved to obtain a heat stress relieving agent in the form of soft drinks. This product is a heat stress relieving agent that exhibits perspiration accelerating effect or suppressing effect on rise in core body temperature and has a good flavor and taste.

### Example 4

### <Heat stress relieving agent in hard candy form>

The mixture of 10 parts by mass of α,α-trehalose dihydrate crystal-containing powder (product name "TREHA", sold by Hayashibara Co., Ltd.) and 20 parts by mass of water was concentrated under reduced pressure at 155°C until the water content became about 2% or less. Then, to this concentrate, 0.5 parts by mass of citric acid, 0.5 parts by mass of α-glucosyl hesperidin-containing powder (product name "Hayashibara Hesperidin S", sold by Hayashibara Co., Ltd.), 0.01 parts by mass of sodium chloride and an appropriate amount of lemon flavor were mixed, and was formed and packaged according to the conventional method to obtain a heat stress relieving agent in the form of hard candy. This product is a heat stress relieving agent that exhibits perspiration accelerating effect or suppressing effect on rise in core body temperature and has a good flavor and taste.

### Example 5

### <Heat stress relieving agent in chewing gum form>

Three parts by mass of a gum base was heated and melted until soft, and 6 parts by mass of α, α-trehalose (product name "TREHA", sold by Hayashibara Co., Ltd.) and 1 part by mass of glycosyl hesperetin-containing powder (a-glucosyl hesperidin 82% by mass, hesperidin 1% by mass, 7-O-β-glucosyl hesperetin 8% by mass, and other components 9% by mass) (prepared by Hayashibara Co., Ltd.) were added and, further 0.1 parts by mass of citric acid and an appropriate amount of a coloring agent, and a flavor were mixed respectively, and then kneaded and molded by a conventional method and packaged to obtain a chewing gum containing α-glucosyl hesperidin. This product is a heat stress relieving agent that exhibits perspiration accelerating effect or suppressing effect on rise in core body temperature and has a good flavor and texture.

### Example 6

### <Heat stress relieving agent in oral tube feeding nutrition form >

A mixture of 30 parts by mass of maltose (product name "Maltose PH", sold by Hayashibara Co., Ltd.), 3 parts by mass of monosodium glutamate, 2 parts by mass of glycine, 2 parts by mass of sodium chloride, 2 parts by mass of citric acid, 0.3 parts by mass of magnesium carbonate, 1 part by mass of calcium lactate, 5 parts by mass of α-glucosyl hesperidin crystal powder (purity 99 % by mass or higher, prepared by Hayashibara Co., Ltd.), 0.02 parts by mass of thiamine and 0.02 parts by mass of riboflavin, was prepared. The mixture was divided into 30 g portions in laminated aluminum bags and heat-sealed to prepare a heat stress relieving agent in tube feeding or oral feeding nutrition form. This product is a heat stress relieving agent that exhibits perspiration accelerating effect or suppressing effect on rise in core body temperature, when dissolved in about 250 to 500 mL of sterile water per bag, the product can be used advantageously as a tube-feeding or oral feeding liquid to improve and prevent heat illness.

### Example 7

### <Heat stress relieving agent in capsule form>

Ten parts by mass of calcium acetate monohydrate, 50 parts by mass of magnesium L-lactate trihydrate, 57 parts by mass of maltose, 20 parts by mass of α-glucosyl hesperidin-containing powder (product name "Hayashibara hesperidin S", sold by Hayashibara Co., Ltd.), 12 parts by mass of γ-cyclodextrin inclusion compound containing 20% vitamin E, and 1 part by mass of ginger extract powder were uniformly mixed, and granules were made by a granule molding machine. The resulting granules were then encapsulated in gelatin capsules according to the conventional method to obtain a heat stress relieving agent in capsule form, which contained 150 mg of content per capsule. This product exhibits perspiration accelerating effect or suppressing effect on rise in core body temperature and can be used advantageously as capsules for improving and preventing heat illness.

### Example 8

### <Heat stress relieving agent in powder form>

Ten parts by mass of glycosyl hesperetin-containing powder (82% by mass of α-glucosyl hesperidin, 1% by mass of hesperidin, 8% by mass of 7-O-β-glucosyl hesperetin, and 9% by mass of other ingredients) (prepared by Hayashibara Co., Ltd.), three parts by mass of sucrose, one part by mass of maltitol and 15 parts by mass of water soluble dietary fiber (product name "Fibryxa", sold by Hayashibara Co., Ltd.) were stirred and mixed. The mixture was then divided into stick-shaped light and moisture-proof packaging of 5 g each to obtain a heat stress relieving agent in powder form. The product exhibits perspiration-promoting effect or suppressing effect on rise in core body temperature, and which can be taken orally on a daily basis by adding one or two sticks of it into various beverages such as water, tea, black tea, coffee or other food and beverages.

### Example 9

### <Heat stress relieving agent in tablet form>

Ten parts by mass of L-ascorbic acid, 19 parts by mass of α-glucosyl hesperidin crystal powder (purity 99 mass% or more, prepared by Hayashibara Co., Ltd.), 70 parts by mass of maltose (product name "Sunmalt", sold by Hayashibara Co., Ltd.), and 1 part by mass of sucrose stearate were uniformly mixed and tableted according to the conventional method to obtain a heat stress relieving agent in the form of tablets. This product is useful as a heat stress reliever that exhibits perspiration accelerating effect or suppressing effect on rise in core body temperature and has a good flavor and texture. This product is useful as a health food for beauty care because it has a swelling-improving effect, a dry skin-improving effect, a metabolism-promoting effect, and a detoxifying effect due to its excellent perspiration accelerating effect.

### Example 10

### <Heat stress relieving agent in gummy candy form>

One hundred and fifty parts by mass of a commercially available α-maltosyl trehalose-containing syrup (product name "Hallodex", sold by Hayashibara Co., Ltd.) was heated under reduced pressure and concentrated to a water content of 15% by mass. To this concentrate, 13 parts by mass of gelatin dissolved in 18 parts by mass of water according to the usual method, 2 parts by mass of α-glucosyl hesperidin-containing powder (product name "Hayashibara Hesperidin S", sold by Hayashibara Co., Ltd.), 2 parts by mass of citric acid, 0.1 parts by mass of anhydrous crystal powder of ascorbic acid 2-glucoside (product name "Ascofresh", sold by Hayashibara Co., Ltd.), 0.1 parts by mass of sodium chloride, and an appropriate amount of coloring and flavoring agents, were uniformly mixed and was formed and packaged according to the conventional method to obtain a heat stress relieving agent in the form of gummy candy. This product is useful as a heat stress reliever that exhibits perspiration accelerating effect or suppressing effect on rise in core body temperature and has a good flavor and texture. This product is useful as a health beauty food because it has a swelling-improving effect, a dry skin-improving effect, a metabolism-promoting effect, and a detoxifying effect due to its excellent perspiration accelerating effect.

### Example 11

### <Heat stress relieving agent in supplement form>

One part by mass of glycosyl hesperetin-containing powder (82% by mass of α-glucosyl hesperidin, 1% by mass of hesperidin, 8% by mass of 7-O-β-glucosyl hesperetin, 9% by mass of other ingredients) (prepared by Hayashibara Co., Ltd.), 50 parts by mass of water-soluble dietary fiber (product name "Fibryxa", sold by Hayashibara Co., Ltd.), 10 parts by mass of α,α-trehalose dihydrate crystal-containing powder (product name "TREHA", sold by Hayashibara Co., Ltd.), 1 part by mass of glycine, 0.1 part by mass of theanine, 0.1 part by mass of GABA, and 0.1 mass part of carnitine were uniformly mixed and filled 3 g each into a stick-shaped paper package whose inner surface was coated with aluminum foil to obtain a heat stress relieving agent in supplement form. This product is useful as a heat stress relieving agent that exhibits perspiration accelerating effect or suppressing effect on rise in core body temperature and has a good flavor and taste. This product is also useful as a health food because it has a restful sleep effect, a sleep-inducing effect, and a sleep-improving effect due to its excellent suppressing effect on rise in core body temperature.

### Example 12

### <Heat stress relieving agent in yogurt form>

The raw material for fermented milk (yogurt mix) was prepared by mixing 30 parts by mass of a α-glucosyl hesperidin crystal powder (purity 99% by mass or higher, prepared by Hayashibara Co., Ltd.), 700 parts by mass of a skimmed milk powder, and 4,000 parts by mass of purified water. This was heat sterilized at 95°C for 10 minutes, and then cooled to 45°C. Then, 100 g of a mixed starter was inoculated and fermented in a tank at 47°C for 5 hours, and then cooled to below 8°C to obtain a heat stress relieving agent in yogurt form. This yogurt is useful as a heat stress relieving agent that exhibits perspiration accelerating effect or suppressing effect on rise in core body temperature and has a good flavor and taste, and can relieve or ameliorate symptoms and effectively delay or prevent the onset of heat illness by allowing patients who develop heat illness or humans who are concerned about the onset of heat illness to routinely ingest 100 to 500g of product, usually per day of adulthood. This product is also useful as a health food because it has a restful sleep effect, a sleep-inducing effect, and a sleep-improving effect due to its excellent suppressing effect on rise in core body temperature.

### Example 13

### <Basal body temperature maintaining agent in powder form>

Ten parts by mass of α-glucosyl hesperidin-containing powder (product name "Hayashibara Hesperidin S", sold by Hayashibara Co., Ltd.), 9 parts by mass of α,α-trehalose dihydrate crystal-containing powder (product name "TREHA", sold by Hayashibara Co., Ltd.), 1 part by mass of lactosucrose (product name "Nyukaoligo", sold by Hayashibara Co., Ltd.), 30 parts by mass of dextrin, 0.1 parts by mass of powder containing anhydrous crystal of ascorbic acid 2-glucoside (product name "Ascofresh", sold by Hayashibara Co., Ltd.) and 0.1 parts by mass of coenzyme Q10 were stirred and mixed, and 5 g of the mixture was divided into a light and moisture-proof packaging in the form of a stick to obtain a basal body temperature maintaining agent in powder form. This product exhibits a basal body temperature maintaining effect, and one or two sticks of this product per day can be added to various beverages such as water, tea, black tea, coffee and other food and beverages, and can be taken orally on a daily basis.

### Example 14

### <Basal body temperature maintaining agent in mixed sweetener form>

One hundred parts by mass of isomerized liquid sugar, 1 part by mass of α,α-trehalose dihydrate crystal-containing powder (product name "TREHA", sold by Hayashibara Co., Ltd.), 1 part by mass of lactosucrose (product name "NyukaOligo", sold by Hayashibara Co., Ltd.), 1 part by mass of glycosyl hesperetin-containing powder (82% by mass of α-glucosyl hesperidin, 1% by mass of hesperidin, 8% by mass of 7-O-β-glucosyl hesperetin, 9% by mass of other ingredients) (prepared by Hayashibara Co., Ltd.) were heat-treated at 80 °C for 30 minutes to obtain a basal body temperature maintaining agent in the form of a mixed sweetener. This product is useful as a liquid-type mixed sweetener with an excellent basal body temperature maintaining effect, because it has good flavor and taste, and can be easy to cook in various foods and beverages.

### Example 15

### <Basal body temperature maintaining agent in health supplemental beverage form>

Forty parts by mass of isomerized sugar, 2 parts by mass of maltitol, 10 parts by mass of black vinegar, 5 parts by mass of apple vinegar, 2 parts by mass of citric acid, 2 parts by mass of malic acid, 2 parts by mass of concentrated apple juice, 2 parts by mass of α-glucosyl hesperidin crystal powder (purity 99% by mass or more, prepared by Hayashibara Co., Ltd.) were mixed and dissolved to prepare a basal body temperature maintaining agent in the form of a health supplemental beverage. This product has a good flavor and taste, which is useful as a basal body temperature maintaining agent.

### Example 16

### <Basal body temperature maintaining agent in powder form>

Ten parts by mass of α-glucosyl hesperidin-containing powder (product name "Hayashibara Hesperidin S", sold by Hayashibara Co., Ltd.), 3 parts by mass of α, α-trehalose dihydrate crystal-containing powder (product name "TREHA", sold by Hayashibara Co., Ltd.), 1 part by mass of maltitol, 15 parts by mass of water-soluble dietary fiber ("Fibryxa" (registered trademark), sold by Hayashibara Co., Ltd.), 0.1 part by mass of royal jelly (product name "Hayashibara Royal Jelly", sold by Hayashibara Co., Ltd.), 0.1 part by mass of soy isoflavone and 0.1 part by mass of placenta extract were stirred and mixed, and 5 g of the mixture was divided into a light and moisture-proof packaging in the form of a stick to obtain a basal body temperature maintaining agent in powder form. This product is useful as a health beauty food having an effect of improving cold symptoms and menopausal disorders due to an excellent in basal body temperature maintaining effect.

### Example 17

### <Basal body temperature maintaining agent in tablet form>

Twentynine parts by mass of glycosyl hesperetin-containing powder (82% by mass of α-glucosyl hesperidin, 1% by mass hesperidin, 8% by mass of 7-O-β-glucosyl hesperetin, 9% by mass of other ingredients) (prepared by Hayashibara Co., Ltd.), 70 parts by mass of maltose (product name "Sunmalt", sold by Hayashibara Co., Ltd.), and 1 part by mass of sucrose stearate, 0.1 part by mass of garlic extract, 0.1 part by mass of maca extract, 0.1 part by mass of oyster extract, 0.1 part by mass of zinc, 0.05 part by mass of arginine, 0.05 part by mass of citrulline were uniformly mixed and then tableted according to the conventional method to obtain a basal body temperature maintaining agent in tablet form. This product is useful as a health beauty food having an effect for maintaining basal body temperature, and thus has an effect of improving cold symptoms and menopausal disorders due to excellent in basal body temperature maintaining effect.

### INDUSTRIAL APPLICABILITY

As described above, the present invention relates to a heat stress relieving agent containing glycosyl hesperetin as an effective ingredient, which is based on the original finding that glycosyl hesperetin exerts a remarkable perspiration acceleration effect and a core body temperature rise suppressing effect in humans. Further, the present invention relates to a basal body temperature maintaining agent containing glycosyl hesperetin as an effective ingredient based on an original finding that glycosyl hesperetin exerts a remarkable basal body temperature maintaining effect in humans. The heat stress relieving agent or the basal body temperature maintaining agent of the present invention is effective in maintaining and recovering health when used regularly by healthy or sick people. In particular, the heat stress relieving agent of the present invention can be used as a perspiration accelerator or suppressor of rising core body temperature, and has the excellent feature of exerting the desired effect more remarkably under so-called heat stress environments such as summer season or during laboring, when it is applied to the subjects who are concerned about heat illness, it is more effective than other subjects. Furthermore, the basal body temperature maintaining agent of the present invention has an excellent feature of exerting the desired effect more remarkably, when applied to the subjects who are concerned about body temperature dysregulation due to chronic sensitivity to cold and menopause disorder or the like, in addition to under so-called cold stress environments, such as winter season or cooling.

### DESCRIPTION OF SYMBOLS

In Figure 1,
○: Placebo sample ingestion group
■: Test sample ingestion group

In Figure 2,
•: High basal body temperature group
■: Low basal body temperature group
*: significant difference at a significance level (p-value) of less than 0.05 (risk rate: less than 5%).
**: significant difference at a significance level (p-value) of less than 0.01 (risk rate: less than 1%).

## Claims

1. A heat stress relieving agent comprising glycosyl hesperetin as an effective ingredient.

2. The heat stress relieving agent according to claim 1 as a perspiration accelerator in a heat stress environment.

3. The heat stress relieving agent according to claim 1 as a suppressor of rising core body temperature in a heat stress environment.

4. The heat stress relieving agent according to any one of claims 1 to 3, wherein said glycosyl hesperetin is 1 or more selected from hesperidin, α-glycosyl hesperidin, and 7-O-β-glucosyl hesperetin.

5. The heat stress relieving agent of claim 4, wherein said α-glycosyl hesperidin is a α-glucosyl hesperidin.

6. A basal body temperature maintaining agent comprising glycosyl hesperetin as an effective ingredient.

7. The basal body temperature maintaining agent according to claim 6, wherein said glycosyl hesperetin is 1 or more selected from hesperidin, α-glycosyl hesperidin, and 7-O-β-glucosyl hesperetin.

8. The basal body temperature maintaining agent of claim 7, wherein said α-glycosyl hesperidin is a α-glucosyl hesperidin.
